# EUROPEAN PATENT SPECIFICATION

(11) **EP 2 351 758 B1**
(45) Date of publication and mention of the grant of the patent: **10.05.2017**
(21) Application number: 08877097.9
(22) Date of filing: 05.12.2008
(51) Int. Cl.: C07F 5/00, C07F 13/00, A61P 35/04, A61K 51/02, C07F 9/6506, A61K 51/04

(54) **METAL COMPLEXES, A METHOD FOR PREPARING SAME, RADIOPHARMACEUTICAL MEANS BASED THEREON**
METALLKOMPLEXE, VERFAHREN ZU DEREN HERSTELLUNG UND DARAUF BASIERENDE RADIOPHARMAZEUTISCHE MITTEL
COMPLEXES MÉTALLIQUES, PROCÉDÉ DE PRODUCTION DE CES DERNIERS, ET AGENTS RADIO-PHARMACEUTIQUES LES UTILISANT

(30) Priority: 29.09.2008 RU 2008138587
(43) Date of publication of application: 03.08.2011
(73) Proprietor: Zakrytoe Aktsionernoe Obschestvo "Farm-Sintez", Moscow 117312 (RU)
(72) Inventor: MALYSHEVA, Anna Olegovna, Moscow 125445 (RU); KLEMENTYEVA, Olga Evgenievna, Moscow 123592 (RU); KODINA, Galina Evgenievna, Moscow 123592 (RU); KORSUNSKY, Valentin Nikolaevich, Moscow 127369 (RU); NAZARENKO, Mihail Evgenievich, Moscow 113191 (RU); MIHAYLOV, Oleg Rotislavovich, Moscow 129642 (RU); PERMINOV, Sergei Vladimirovich, Moscow 109444 (RU); UVAROV, Nikolai Aleksandrovich, Moscow 127410 (RU); FEDOROV, Vladimir Egorovich, Moscow 125028 (RU)
(74) Representative: Held, Stephan
(86) International application number: PCT/RU2008/000748
(87) International publication number: WO 2010/036140

(56) References cited:
- EP-A1- 1 925 621
- EP-A2- 1 698 351
- RU-C1- 2 045 282
- RU-C2- 2 297 229
- US-A- 5 772 982
- DENG-KE CAO ET AL: "Copper diphosphonates with zero-, one- and two-dimensional structures: ferrimagnetism in layer compound Cu3(ImhedpH)2.2H2O [ImhedpH4 = (1-C3H3N2)CH2C(OH)(PO3H2)2]", DALTON TRANSACTIONS, no. 37, 1 January 2008 (2008-01-01), page 5008, XP55018494, ISSN: 1477-9226, DOI: 10.1039/b805487h
- WANG H., LUO S., XIE M., LIU X.: "The new bone-imaging agent: preparation and biodistribution of 99Tcm-ZL", NUCLEAR TECHNIQUES, vol. 29, 2006, pages 438-441, XP009156348,
- NEVES M ET AL: "Synthesis, characterization and biodistribution of bisphosphonates Sm-153 complexes: correlation with molecular modeling interaction studies", NUCLEAR MEDICINE AND BIOLOGY, ELSEVIER, NY, US, vol. 29, no. 3, 1 April 2002 (2002-04-01), pages 329-338, XP004346639, ISSN: 0969-8051, DOI: 10.1016/S0969-8051(01)00305-5
- MARCZEWSKI B. ET AL.: "Liquid Kit for Preparation of 188Rhenium-etidronate", BRAZILIAN ARCHIVES OF BIOLOGY AND TECHNOLOGY, vol. 48, October 2005 (2005-10), pages 137-140, XP002669159,

## Description

The invention relates to the chemistry and medicine, in particular, to the metal complexes, method of their preparation, radiopharmaceutical compositions on their basis and method of their production, and can be used for the diagnostics and treatment in the oncology, namely, in the therapy of the myelogenic disease, osseous metastases at the mammary gland, lungs, thyroid, colon, uterine body, prostate carcinomas, and as a mean to reduce the hypercalcemia.

The complexes of radionuclides with the di- and poly-phosphonates have the ability to selectively accumulate in the skeleton, especially in the pathologic osteogenesis zones. The main advantage of the skeleton scintigraphy compared with the traditional X-ray diagnostics is the early detection of both the single and multiple loci of increased accumulation at the malignant neoplasms of the osseous skeleton and bone metastases.

Currently known are the ^{99m}Tc phosphate complexes, in particular the polyphosphate, triphosphate, and pyrophosphate [G.Subramanian, J.G.Agfee//Radiology-1971.-Vol.99.-p.192].

Currently known are also the diphosphonates ^{99m}Tc, with similar properties [Schmitt G.H., Holmes R.A., Isitman AT. //Radiology-1974.-Vol.112.- p.733].

As of today, the world radiological practice uses essentially the ^{99m}Tc-methylenediphosphonate (^{99m}Tc-MDP) and ^{99m}Tc-hydroxyethylidene-diphosphonate (^{99m}Tc-HEDP) [W.C.Eckelman, W.A.Volkert. //Int.J.Appl.Radiat.Isotopes-1982.-Vol.33.-p.945]. To receive the ^{99m}Tc the pertechnetate, sodium ^{99m}Tc, obtained from the ⁹⁹Mo/^{99m}Tc generator is used. The Tc(VII) is the most chemically stable state of the technetium; however, the pertechnetate ions do not combine with the chelating agents. To receive the stable compounds of the reduced technetium different reducers are needed, such as the tin ions (II), sodium boron hydride, concentrated hydrochloric acid, sodium dithionite, and hydrazine [Nuclear Medicine. Diagnosis and Therapy//J.C.Harbert, W.C.Eckelman, R.D.Neumann Eds.-Thieme Medical Publishers, Inc., New York, 1990, p. 218]. As of the reducer, the tin dichloride that is a constituent of practically all the ^{99m}Tc preparations is the most widely used. As the tin dichloride is in a great excess in relation to the ^{99m}Tc, it supports the technetium in the reduced form and supports the formation of the reduced technetium complex with the ligands.

All the technetium complexes with the diphosphonates are osteotropic; hence, the "^{99m}Tc-phosphonate complex" is a standard formulation.

Alongside with the ^{99m}Tc, the radionuclide therapy uses the rhenium and samarium isotopes, in particular, the ¹⁵³Sm-ethylenediaminetetramethylenephosphate (¹⁵³Sm-EDTMP) [Resche I.,Catal J.F.,Peching A. //Eur. J/ Cancer. - 1997. -Vol.33, p.1583], samarium-153-oxabifor [Krylov V.V., Tsyb A.F.,Drosdovsky B.Y. //Eur.J.Nucl.Med. and Molec.Imaging.-2006.- Vol.33, Suppl. 2.- s. 335], rhenium-186- and rhenium-188-hydroxyethylenediphosphonate (¹⁸⁶Re-HEDP [Jak M.S.P., Han S.H. Zonnenberg B.A, et al.//J.Nucl.Med.- 1996.-Vol.37.- p.1511],¹⁸⁸Re-HEDP [Faintuch B.L.Faintuch S., Muramoto E. // Radiochim.Asta- 2003.- Vol. 91.- p.607], ¹⁸⁸-Re-methylenediphosphonate (¹⁸⁸Re-MDP) [Hashimoto K., Bagiawati S.Jzumo M., Kobayashi K. //Appl. Radiat. Isot.-1996-Vol. 47, No.2-p.195]).

The rhenium-186 is obtained in a nuclear reactor, in the reaction ¹⁸⁵Re (n,γ) ¹⁸⁶Re, or in a cyclotron, in the reaction ¹⁸⁶W (p, n) ¹⁸⁶Re [Jak M.S.P., Han S.H., Zonnenberg B.A. et al. //J. Nucl. Med.- 1996.-Vol.37.-p. 1511]. These methods are expensive enough. The rhenium-188 generator was developed wherein the parent isotope was the W-188, received from the W-186 concentrated metallic tungsten or tungsten oxide [Knapp F.F. Mirzadeh S., Zamora P.,et al .//Nucl. Med. Commun.-1996.-Vol. 17.-p. 268].

The rhenium, just like the technetium, forms complexes with different ligands. The processes of the rhenium-188 formation with the three methylenephosphonic acids, namely, the ethylenediamine-N,N,N',N'-tetrakis(methylenephosphoric acid, EDTMP); (ethylenediamine-N,N'-bis-methylenephosphoric acid, EDBMP); (nitrilotris-methylenephosphoric acid, NTMP) were studied. It was noted that in presence of the tin dichloride all the ligands form complexes at the pH < 3 [Hashimoto K. The Second Japanese-Russian Seminar on technetium. Shizuoka, Japan, 1999, Abstracts.- p.40.].

Along with the diphosphonates, the medical practice often uses the biphosphonates [Bonevolenskaya L.I. Biphosphonates and Osteoporosis.// Handbook on Osteoporosis/ Ed.by Bonevolenskaya- Moscow, Binosh, Laboratoriya znaniy.-2003.-CH.9.- p.196-216.]. The biphosphonates acute, subacute, and chronic toxicity studies show that they are, in whole, in the group of the low toxicity compounds with the relatively low acute and chronic toxicity levels. Neither have they demonstrated the teratogenic, mutagenic, or carcinogenic properties.

The zoledronic acid is a biphosphonate and can inhibit the osseous resorption. The antiresorptive mechanism is not completely clear. In vitro, the zoledronic acid inhibits the activity and induces the apoptosis of the osteoclasts. It blocks the osteoclastic resorption of the mineralized osseous and cartilaginous tissues.

In Storto G., Paone G., Ibello F., et al. // Eur.J. Nucl. Med. And Molec.Imaging.- 2004.-Vol. 31, - s. 291. the authors applied the combined Sr-89 + zoledronic acid therapy to reduce the osseous pains at the metastases resulting from the mammary gland and prostate gland carcinomas; they compared the results with those of the therapy using only the Sr-89.

A known document by Wang et al (Nuclear techniques, Vol. 29, 2006, p. 438-441, XP 009156348) describes ^{99m}Tc^{m} labeled zoledronic acid complexes obtained by Na^{99m}Tc^{m}O₄ with SnCl₂·▪ 2 H₂O reduction, but it is not detailed therein, that to obtain it, HCl (hydrochloric acid), inert gas, (and possibly) alkali metal hydroxide and lyophilisation are used.

As a result, a zoledronic acid metal complex is obtained, which is different from the metal complex being one of the inventions of the claimed group, which is clearly seen when comparing the properties of the resulting radiopharmaceutical means (hereinafter referred to as "RM") based on these different metal complexes.

As of today, the skeleton scintigraphy (diagnostics) uses, mainly, the simplest technetium-^{99m}-labeled diphosphonates of the general formula: 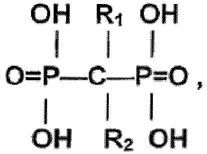 where R₁ and R₂ denote the hydrogen, hydroxy, carboxy, hydrocarbonic radical, e.g., the methylenediphosphonate (MDP), oxyethyldiphosphonate (HEDP) or dicarboxydiphosphonate (OPP) [W.C.Eckelman, W.A.Volkert. //Int.J.Appl.Radiat.Isotopes-1982.-Vol.33.-p.945] and the rhenium-186 or rhenium-188-labeled diphosphonates, mainly the oxyethylidenediphosphonate [Jak M.S.P., Han S.H. Zonnenberg B.A, et al. //J.Nucl.Med.- 1996.-Vol.37.- p.1511], ¹⁸⁸Re-HEDP [Faintuch B.L.Faintuch S., Muramoto E. // Radiochim.Asta- 2003.- Vol. 91.- p.607].

However, their specificity, diagnostic sensitivity, and treatment efficiency are not sufficiently high. Further, Wang et al. (Nuclear techniques, vol. 29, 2006, p. 438-441, XP009156348) describes ⁹⁹Tc^{m} labeled zoledronate (ZL), prepared by the reduction of Na⁹⁹Tc^{m}O₄ with SnCl_{2▪}2H₂O, studying its biodistribution in mice and the bone scan in rabbits respectively, revealing the ⁹⁹Tc^{m}-ZL as a potential bone-imaging agent.

The purpose of this invention is to eliminate the above-listed limitations.

The objective is reached by the inventions relative to the proposed metal complexes, the method of their preparation, the radiopharmaceuticals on basis of the metal complexes and the method of production of the radiopharmaceutical.

The formula of the proposed metal complexes is the following:

M(O)ₖHₘAⁿ⁻,

obtained with the use of the proposed method, where M is an isotope of Tc, Re, Sm, Lu or Y, H is hydrogen, Aⁿ⁻ is the anion of zoledronic acid,
wherein, when M is technetium, k=1, m=1, n=2; when M is rhenium, k=1, m=1, n=1; when M is samarium, lutetium, or yttrium,
k=0, m=1, n=1 and have the pH value 2-6 and radiochemical purity ≥ 90-95%, as well as
their pharmaceutically acceptable salts, hydrates or isomers.

The metals isotopes are chosen from the group consisting from ^{186,188}Re, ^{99m}Tc, ¹⁵³Sm, ¹⁷⁷Lu, ⁹⁰Y, as well as their pharmaceutically acceptable salts, hydrates or isomers, wherein the preferable metals isotopes are the ^{186,}188Re, ^{99m}Tc.

The reducing agent is tin dichloride in hydrochloric acid.

The proposed method of preparation of the metal complex is characterized in that the lyophilizate, obtained by mixing the solution of zoledronic acid and the solution of tin dichloride in hydrochloric acid in an inert-gas atmosphere and a possible addition of an alkaline metal hydroxide, is mixed with the salt of an isotope group metal and a radionuclide solution.

The proposed radiopharmaceutical contain the metal complexes, containing radioactive isotopes ^{99m}Tc, ¹⁸⁶Re, ¹⁸⁸Re, with the reducing reagent of tin dichloride in hydrochloric acid, as well as, when required, an alkali metal hydroxide, a metal of the group of isotopes and an antioxidant, either the ascorbic acid or the gentisinic acid.

The production process of the radiopharmaceutical comprise mixing a solution of zoledronic acid and a solution of tin dichloride in hydrochloric acid in an inert-gas environment and an addition of an alkali metal hydroxide when required, obtaining of the lyophilizate and the consequent mixing thereof with the solution of radioactive ^{99m}Tc. In a further embodiment the production of radiopharmaceuticals comprise mixing a solution of zoledronic acid in an inert-gas environment, obtaining of lyophilizate and the consequent mixing thereof with the solution of the radionuclide ¹⁸⁶Re or ¹⁸⁸Re.

Wang et al states that phosphonic acid salts (hereinafter referred to as "PAS") are used for buffering, which leads to ^{99m}Tc^{m} competitive interaction with both zoledronic acid (hereinafter referred to "ZA") and PAS. As a result, an entirely different metal complex is formed, which does not provide for sufficient volumetric ZA labeling activity, which reduces its biological activity. The metal complex of the present invention possesses high biological activity and provides for increased efficiency of radiopharmaceutical means (hereinafter referred to as "RM") obtained on the basis of the aforesaid metal complex containing zoledronic acid. RM effectiveness as a bone scan diagnostic agent - skeletal retention of RM - amounts to 47.3% of the dose injected 3 hours after the administration thereof, while in the known solution (Wang et al) the aforesaid indicator is considerably lower and amounts to 34.2%. In the present invention considerable differences are observed in biodistribution and delayed retention indices (hereinafter referred to as "DRI") for RM and RM using the prior metal complex. When RM is administered in the present invention, 3 hours after administration the retention level thereof in the liver amounts to as little as 1.0 % of the doze injected, while in case of the administration of the RM containing the prior metal complex, the retention level thereof in the liver amounts to 13.4 % of the doze injected (3 hours after administration). Increased retention of diagnostic RM in the liver is a known unfavorable factor affecting the quality of radioisotope diagnostics. All aforesaid advantages of the invention claimed are technical advantages. The examples below illustrate the production of the proposed complexes and radiopharmaceutical.

### Example 1:

### Synthesis of the preparation with the technetium-^{99m}:

### 1. Preparation of the lyophilized reagent

0.15 grams of the zoledronic acid were dissolved in 92 ml of water in an inert gas flow. This and following operations were performed in an inert gas flow. To the resulting solution, 5 ml of the tin dichloride solution in 1 M hydrochloric acid (6-7 mg/ml) and 3 ml of the 1.5 M NaOH solution were added. The resulting reagent solution was passed through the 0.22 µm filter and packed in the medicinal vials by 1 ml portions. The vials contents were lyophilized.

### 2. Composition of lyophilizate in vial:

| | |
|---|---|
| zoledronic acid | 1.53 mg |
| tin dichloride, anhydrous | 0.3 mg |
| pH | 4-6,5 |

### 3. Preparation

In the lyophilized reagent vial, 5 ml of the isotonic solution of the sodium pertechnetate, ^{99m}Tc (740-1480 MBq/ml) from the generator were added; this was cured 20 minutes under the room temperature.

### 4. Composition of preparation:

| | |
|---|---|
| Technetium-^{99m} in form of complex | |
| with zoledronic acid | 740-1480 MBq/ml |
| zoledronic acid | 0.31 mg/ml |
| tin dichloride, anhydrous | 0.06 mg/ml |
| pH | 4-6 |
| Radiochemical purity | > 95 % |

(determined by the HELC and TLC methods)

### Example 2:

### Synthesis of preparation with the rhenium-188

### 1. Preparation of the lyophilized reagent

0.42 grams of the gentisinic acid were dissolved in 60 ml of water. This and following operations were performed in an inert gas flow. To the resulting solution, 8 ml of the tin dichloride solution in 1 M hydrochloric acid (18 mg/ml) and 40 ml of the zoledronic acid aqueous solution (6 mg/ml) were added. The resulting reagent solution was passed through the 0.22 µm filter and packed in the medicinal vials by 1 ml portions. The vials contents were lyophilized.

### 2. Composition of lyophilized reagent in vial:

| | |
|---|---|
| zoledronic acid | 2.2 mg |
| tin dichloride | 1.33 mg |
| gentisinic acid | 3.9 mg |
| pH | 2,4 |

### 3. Preparation:

a) Into the vial with 40 µl of the sodium perrhenate of the 0.8 mg/ml rhenium concentration, 1.5 ml of the sodium perrhenate, ¹⁸⁸Re, solution was added. The solution was stirred (vial No 2).
b) Into the vial with the lyophilized reagent (vial No 1), all the content of the vial No 2 was transferred with the help of a syringe; then it was stirred.
c) The preparation was kept under room temperature within 2 h. Before use, the preparation solution was passed through the 0.22 µm filter.

### 4. Composition of preparation:

| | |
|---|---|
| rhenium-188 in form of complex | |
| with zoledronic acid | 740-7400 MBq/ml |
| zoledronic acid | 1.45 mg/ml |
| tin dichloride | 0.89 mg/ml |
| gentisinic acid | 2.59 mg/ml |
| rhenium | 0.02 mg/ml |
| pH | 2-4 |
| Radiochemical purity | > 90 % |

(determined by the HELC and TLC methods)

The other metal complexes are produced similarly to cases 1 and 2.

### Biological testing of proposed metal complexes

The experiments were run on the intact scrub albino rats with modeled osseous pathology. The osseous pathology was imitated by the femur fracture.

To prepare the composition, 5.0 ml of the sodium pertechnetate ^{99m}Tc from the technetium generator (FSP 42-0225-4528-03, FSP 42-0018-2694-02) were injected into the lyophilizate vial by puncturing the rubber stopper with a syringe needle; then the vial was agitated and cured 20 minutes. The 0.2 ml volume of the composition was injected into the caudal vein. 1, 3, 5, and 24 hours after injection the animals were devitalized by decapitation; then the samples of blood, liver, kidneys, stomach, right and left femurs, urinary bladder with contents were taken. The radionuclide in organs and tissues was determined by the direct radiometry.

The delayed retention index was determined as the ratio of division of the composition concentration in the femoral bone with the modeled osseous pathology and in the healthy femoral bone.

The statistical treatment used the Student's method.

The results are given in Tables 1 and 2.

**Table 1 Pharmacokinetics of "zoledronic acid, ^{99m}Tc" in organs and tissues of intact female rats after intravenous introduction (% dose/organ).**

| Organ/tissue | 1 h | 3 h | 5 h | 24 h |
|---|---|---|---|---|
| Blood | 1.7 ± 0.27 | 0.9 ± 0.38 | 0.4 ± 0.03 | traces |
| Liver | 0.8 ± 0.12 | 1.0 ± 0.27 | 0.6 ± 0.11 | 0.2 ± 0.13 |
| Kidneys | 1.3 ± 0.17 | 0.9 ± 0.21 | 1.2 ± 0.13 | 0.6 ± 0.32 |
| Stomach | 0.1 ± 0.03 | 0.1 ± 0.01 | 0.1 ± 0.04 | 0.3 ± 0.05 |
| Urinary bladder (1, 3 h); | 45.4 ± 4.03 | 30.1 ± 4.41 | | |
| Excretion (5, 24 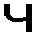 ) | | | 52.4 ± 2.80 | 39.1 ± 5.00 |
| Femur | 1.8 ± 0.16 | 1.9 ± 0.35 | 1.6 ± 0.11 | 1.8 ± 0.06 |
| Skeleton | 48.1 ± 4.36 | 47.3 ± 2.89 | 42.9 ± 2.93 | 49.6 ± 1.77 |

**Table 2 Pharmacokinetics of "zoledronic acid, ^{99m}Tc" in organs and tissues of female rats with modeled osseous pathology after intravenous introduction (% dose/organ).**

| Organ/tissue 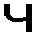 | 1 h | 3 h | 5 h | 24 h |
|---|---|---|---|---|
| Blood | 1.7 ± 0.71 | 2.0 ± 0.81 | 0.5 ± 0.01 | 0.7 ± 0.01 |
| Liver | 1.7 + 0.58 | 1.1 ± 0.42 | 1.0 ± 0.05 | 0.5 ± 0.09 |
| Kidneys | 1.8 ± 0.83 | 1.4 ± 0.68 | 0.9 ± 0.06 | 0.5 ± 0.08 |
| Stomach | 0.4 ± 0.03 | 0.2 ± 0.09 | 0.4 ± 0.05 | traces |
| Urinary bladder (1, 3 h); | 35.1 ± 10.53 | 44.8 ± 3.89 | | |
| Excretion (5, 24 ) | | | 43.4 ± 5.05 | 52.5 ± 10.86 |
| Femur, normal | 2.1 ± 0.20 | 1.9 ± 0.42 | 1.9 ± 0.20 | 1.4 ± 0.40 |
| Femur, fractured | 4.2 ± 1.04 | 4.0 ± 1.34 | 3.1 ± 0.70 | 2.5 ± 0.05 |
| Skeleton | 57.7 ± 5.54 | 46.6 ± 7.81 | 49.9 ± 5.44 | 38.0 ± 11.02 |
| Delayed retention index | 2.0 | 2.1 | 1.7 | 1.9 |

The composition pharmacokinetics study has shown that the composition distribution is characterized by the apparent osteotropicity. After the intravenous injection of the composition in the intact animals, in one hour, up to 48% of the injected activity localizes in the skeleton and 45% is excreted from the organism with the urine. In 3 hours after introduction the retention in the skeleton is 47%; the excretion is 30%. The activity level in blood quickly reduces; in 5 hours after the study start it is, practically, trace.

The composition supports the bone scintigraphy based on the bone pathology/norm delayed retention indexes. This confirms the functional eligibility of the composition as a radiopharmaceutical in diagnosing the osseous tissue pathologic processes accompanied with the osteoblastic processes.

## Claims

1. Method of preparation of a metal complex with a general formula
M(O)ₖHₘAⁿ⁻,
Where M is an isotope of Tc, Re, Sm, Lu or Y;
H is hydrogen, Aⁿ⁻ is an anion of zoledronic acid,
wherein when M is technetium, k = 1, m = 1, n = 2, when M is rhenium, k = 1, m = 1, n = 1, and when M is samarium, lutetium and yttrium, k = 0, m = 1, n = 1;
**characterized in that** lyophilizate, obtained by mixing solution of zoledronic acid and solution of tin dichloride in hydrochloric acid in an inert-gas atmosphere and a possible addition of an alkali metal hydroxide, is mixed with the salt of isotope group metal and radionuclide solution.

2. Metal complexes with the general formula
M(O)ₖHₘAⁿ⁻,
obtained with the use of method, described in claim 1, where M is an isotope of Tc, Re, Sm, Lu or Y,
H is hydrogen, Aⁿ⁻ is an anion of zoledronic acid,
wherein when M is technetium, k = 1, m = 1, n = 2, when M is rhenium, k = 1, m = 1, n = 1; and when M is samarium, lutetium or yttrium, k = 0, m = 1, n = 1;
and have the pH value 2-6 and radiochemical purity ≥ 90-95%, as well as their pharmaceutically acceptable salts, hydrates or isomers.

3. Metal complexes claimed in 2, where isotopes of metals are chosen from the group consisting from ^{99m}Tc, ¹⁸⁶Re, ¹⁸⁸Re, ¹⁵³Sm, ¹⁷⁷Lu, ⁹⁰Y, as well as their pharmaceutically acceptable salts, hydrates or isomers.

4. Radiopharmaceuticals, which include metal complexes claimed in 2, containing radioactive isotopes ^{99m}Tc, ¹⁸⁶Re, ¹⁸⁸Re, with the reducing reagent of tin dichloride in hydrochloric acid, as well as, when required, an alkali metal hydroxide, a metal of the group of isotopes and an antioxidant.

5. The production process of radiopharmaceuticals claimed in 4 comprising mixing a solution of zoledronic acid and a solution of tin dichloride in hydrochloric acid in an inert-gas environment and an addition of an alkali metal hydroxide when required, obtaining of lyophilizate and the consequent mixing thereof with the solution of radionuclide ^{99m}Tc.

6. The production of radiopharmaceuticals claimed in 4, comprising mixing a solution of zoledronic acid in an inert-gas environment, obtaining of lyophilizate and the consequent mixing thereof with the solution of the radionuclide ¹⁸⁶Re or ¹⁸⁸Re.

## Patentansprüche

1. Verfahren zur Herstellung eines Metallkomplexes der allgemeinen Formel:
M(O)ₖHₘAⁿ⁻,
wobei M ein Tc, Re, Sm, Lu oder Y Isotop ist;
H Wasserstoff ist, Aⁿ⁻ ein Anion der Zoledronsäure ist;
wobei, wenn M Technetium ist, k = 1, m = 1, n = 2 ist,
wenn M Rhenium ist, k = 1, m = 1, n = 1, ist,
und wenn M Samarium, Lutetium oder Yttrium ist, k = 0, m = 1, n = 1 ist;
**dadurch gekennzeichnet, dass** ein Lyophilisat, erhalten durch Mischen einer Zoledronsäurelösung mit Lösung von Zinndichlorid in Salzsäure unter Inertgasatmosphäre und der möglichen Zugabe eines Alkalimetallhydroxids, mit dem Salz aus der Gruppe der Metallisotope und Radionuklidlösung gemischt wird.

2. Metallkomplexe der allgemeinen Formel
M(O)ₖHₘAⁿ⁻,
erhalten nach dem Verfahren gemäß Anspruch 1, wobei M ein Tc, Re, Sm, Lu oder Y Isotop ist;
H Wasserstoff ist, Aⁿ⁻ ein Anion der Zoledronsäure ist;
wobei, wenn M Technetium ist, k = 1, m = 1, n = 2 ist,
wenn M Rhenium ist, k = 1, m = 1, n = 1 ist, und wenn M Samarium, Lutetium oder Yttrium ist, k = 0, m = 1, n = 1 ist,
und die einen pH-Wert von 2-6 und eine radiochemische Reinheit > 90-95% aufweisen, sowie deren pharmazeutisch akzeptablen Salzen, Hydraten oder Isomeren.

3. Metallkomplexe gemäß Anspruch 2, wobei die Metallisotope ausgewählt sind aus der Gruppe, bestehend aus ^{99m}Tc, ¹⁸⁶Re, ¹⁸⁸Re, ¹⁵³Sm, ¹⁷⁷Lu, ⁹⁰Y, sowie deren pharmazeutisch akzeptablen Salzen, Hydraten oder Isomeren.

4. Radiopharmazeutika, umfassend die Metallkomplexe gemäß Anspruch 2, beinhaltend die radioaktiven Isotope ^{99m}Tc, ¹⁸⁶Re, ¹⁸⁸Re, das Reduktionsmittel Zinndichlorid in Salzsäure, gegebenenfalls ein Alkalimetallhydroxid, ein Metall aus der Gruppe der Isotope und ein Antioxidans.

5. Verfahren zur Herstellung der Radiopharmazeutika gemäß Anspruch 4, umfassend das Mischen einer Zoledronsäurelösung und einer Lösung von Zinndichlorid in Salzsäure in Inertgasumgebung, und wenn benötigt unter Zugabe eines Alkalimetallhydroxids, das Erhalten von Lyophilisat und das darauffolgende Mischen davon mit der Lösung des Radionuklids ^{99m}Tc.

6. Verfahren zur Herstellung von Radiopharmazeutika gemäß Anspruch 4, umfassend das Mischen einer Zoledronsäurelösung in Inertgasumgebung, Erhalten von Lyophilisats, und das darauffolgende Mischen davon mit der Lösung des Radionuklids ¹⁸⁶Re oder ¹⁸⁸Re.

## Revendications

1. Méthode de préparation d'un complexe métallique avec la formule générale :
M(O)ₖHₘAⁿ⁻
où M est un isotope de Tc, Re, Sm, Lu ou Y;
H est hydrogène, Aⁿ⁻ est un anion de l'acide zolédronique,
où, si M est technétium, k = 1, m = 1, n = 2, si M est rhénium, k = 1, m = 1, n = 1, si M est samarium, lutécium et yttrium, k = 0, m = 1, n = 1;
**caractérisée par le fait que** le lyophilisat obtenu en mélangeant une solution de l'acide zolédronique et une solution de dichlorure de l'étaine dans l'acide chlorhydrique sous l'atmosphère d'un gaz inerte et une addition possible d'un hydroxyde d'un métal alcalin, est mélangé avec le sel d'un métal du groupe isotopique et une solution de radionucléide.

2. Complexes métalliques avec la formule générale
M(O)ₖHₘAⁿ⁻
obtenus en utilisant la méthode décrit dans la revendication 1, où M est un isotope de Tc, Re, Sm, Lu ou Y,
H est hydrogène, Aⁿ⁻ est un anion de l'acide zolédronique,
où, si M est technétium, k = 1, m = 1, n = 2; si M est rhénium, k = 1, m = 1, n = 1; si M est samarium, lutécium ou yttrium, k = 0, m = 1, n = 1;
et ont un potential hydrogène de à 2-6 et une pureté radiochimique ≥ 90-95 %, ainsi que leurs sels, hydrates ou isomères acceptables au niveau pharmaceutique.

3. Complexes métalliques selon revendication 2, où les isotopes des métaux sont choisis de la groupe composé de ^{99m}Tc, ¹⁸⁶Re, ¹⁸⁸Re, ¹⁵³Sm, ¹⁷⁷Lu, ⁹⁰Y, ainsi que leurs sels, hydrates ou isomères acceptables au niveau pharmaceutique.

4. Préparations radiopharmaceutiques, comprenant des complexes métalliques selon revendication 2 contenant des isotopes radioactifs de ^{99m}Tc, ¹⁸⁶Re, ¹⁸⁸Re, avec un agent réducteur du dichlorurede l'étain dans l'acide chlorhydrique, ainsi que, en cas de besoin, un hydroxyde d'un métal alcalin, un métal du groupe isotopique et un antioxydant.

5. Processus de production des produits radiopharmaceutiques selon revendication 4 comprenant le mélange d'une solution de l'acide zoledronique et d'une solution de dichlorure de l'etain dans l'acide chlorhydrique dans une environment d'un gaz inerte et, en cas de besoin, une addition d'un hydroxyde d'un métal alcalin, obtenant du lyophilisat et son mélange subséquent avec la solution de radionucléide ^{99m}Tc.

6. Production des produits radiopharmaceutiques selon revendication 4 comprenant le mélange d'une solution de l'acide zolédronique dans une environment du gaz inerte en obtenant du lyophilisat et son mélange subséquent avec la solution de radionucléide ¹⁸⁶Re ou ¹⁸⁸Re.
